(19) Europäisches Patentamt
European Patent Office
Office européen des brevets

(11) Publication number: **0 027 685 A2**

# (12) EUROPEAN PATENT APPLICATION

(21) Application number: 80303158.2

(22) Date of filing: 09.09.80

(51) Int. Cl.³: C 07 D 249/08
C 07 D 233/60, C 07 C 69/716
C 07 C 49/12, C 07 C 49/203

(30) Priority: 11.10.79 GB 7935400
29.10.79 GB 7937346
25.03.80 GB 8010071

(43) Date of publication of application:
29.04.81 Bulletin 81/17

(84) Designated Contracting States:
AT BE CH DE FR GB IT LI NL

(71) Applicant: IMPERIAL CHEMICAL INDUSTRIES LIMITED
Imperial Chemical House Millbank
London SW1P 3JF(GB)

(72) Inventor: Worthington, Paul Anthony
22 Boulters Gardens
Maidenhead Berkshire(GB)

(72) Inventor: Sugavanam, Balasubramanyan
11 Kiln Ride
Wokingham Berkshire(GB)

(74) Representative: Alner, Henry Giveen Hamilton et al,
Imperial Chemical Industries Limited Legal Department: Patents Thames House North Millbank
London SWIP 4QG(GB)

## (54) Improved process for preparing imidazole and 1,2,4-triazole derivatives.

(57) An improved process for preparing imidazole and 1,2,4-triazole derivatives comprises the steps of:-

(a) reacting a $\beta$-keto ester $R^1$-CO-$CH_2COOR$ with a $\alpha$-halo ketone $R^2COCH_2X$ where X = halogen,
(b) hydrolysing the diketo ester to a diketone $R^1COCH_2CH_2COR^2$
(c) oxidising the diketone ester to an unsaturated diketone
(d) reacting the unsaturated diketone with imidazole or 1,2,4-triazole
(e) selectively reducing the product of (d) to give a compound of formula:

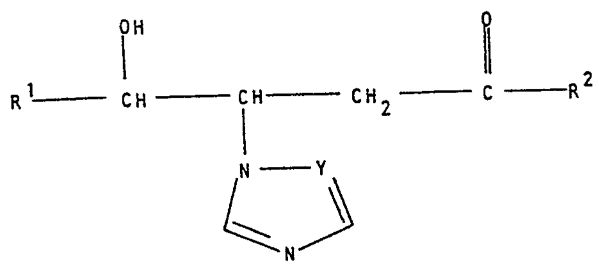

wherein $R^1$ and $R^2$ are optionally substituted alkyl, cycloalkyl or phenyl.

EP 0 027 685 A2

0027685
30994/EP

# IMPROVED PROCESS FOR PREPARING IMIDAZOLE AND 1,2,4-TRIAZOLE DERIVATIVES

## 4-(1,2,4-Triazol-1-yl)-2,2,7,7-tetramethyl-octan-3-ol-6-one

This invention relates to a process for preparing 1,2,4-triazole and imidazole derivatives, and more particularly to a process for preparing the compound 4-(1,2,4-triazol-1-yl)-2,2,7,7-tetramethyloctan-3-ol-6-one.

In our copending U.K. Patent Application No.18861/77 filed on 5 May 1977 (German Offenlegungschrift No. P 28 19 879.0 corresponds) there are described and claimed compounds having the general formula (I)

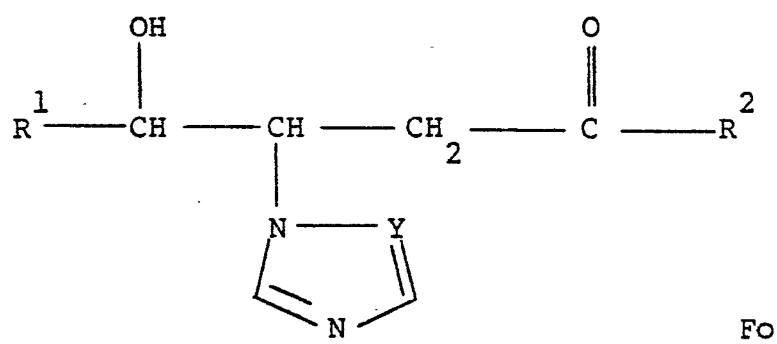

Formula (I)

wherein each of $R^1$ and $R^2$, which may be the same or different, is unsubstituted or alkyl-substituted cycloalkyl, unsubstituted or halo-substituted alkyl, or unsubstituted or substituted phenyl; and Y is =N- or =CH-.

In Example 1 of the specification of the foregoing application there is described a method for the preparation of the compound 4-(1,2,4-Triazol-1-yl)-2,2,7,7-tetramethyl-octan-3-ol-6-one. This compound has useful fungicidal properties and plant growth regulating effects as described in the foregoing application and it is specifically claimed in Claim 8 thereof as a novel chemical compound. The process of Example 1 is specifically claimed in Claim 10 thereof.

The present invention provides an improved process for making the foregoing compound, and compounds of the general formula (Formula I) given above, which results in greatly improved yields of the desired chemical compound; makes use of cheaper starting substances; and uses simpler process reagents and procedures.

Accordingly the invention provides a process for preparing compounds having the general formula (I):

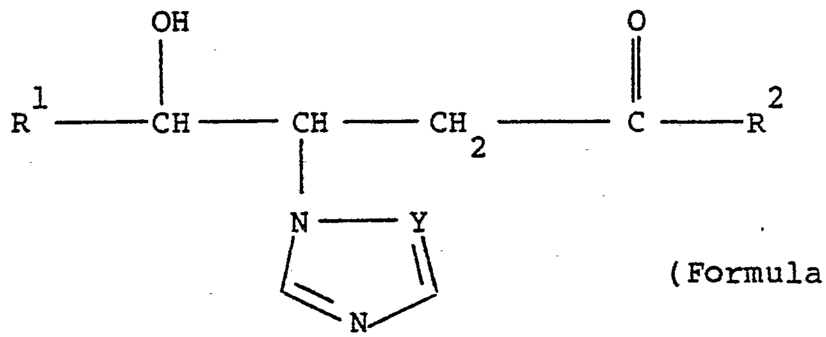

(Formula I)

wherein each of $R^1$ and $R^2$, which may be the same or different, is unsubstituted or alkyl-substituted cycloalkyl, unsubstituted or halo-substituted alkyl, or unsubstituted or substituted phenyl; and Y is =N- or =CH-, which comprises the steps of (a) reacting a β-keto ester of formula:

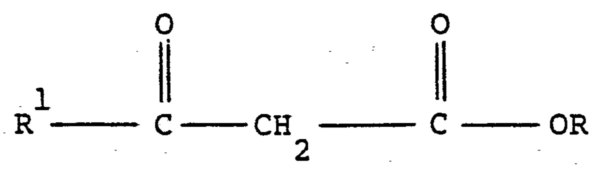

where R is alkyl and especially methyl or ethyl, and $R^1$ is as defined above, with an α-halo ketone of formula:

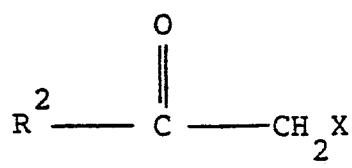

where X is halogen, especially chlorine or bromine, and $R^2$ is as defined above, to produce a diketo-ester of formula:

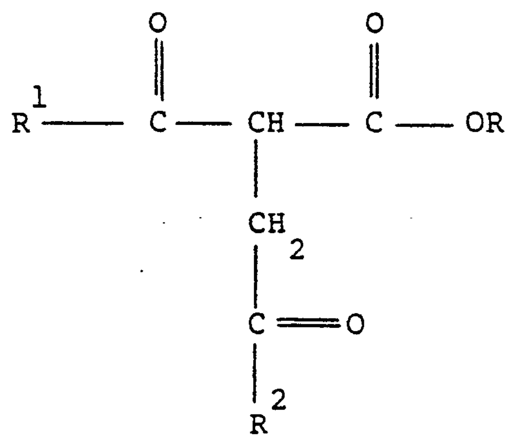

(b) hydrolysing the diketo-ester under basic conditions to give a saturated diketone of formula:

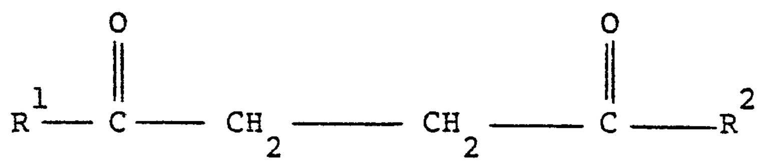

(c) oxidising the saturated diketone to give an unsaturated diketone of formula:

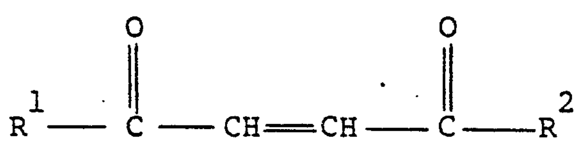

(d) bring the unsaturated diketone (ene-dione) into reaction with 1,2,4-triazole or imidazole to produce the compound of formula:

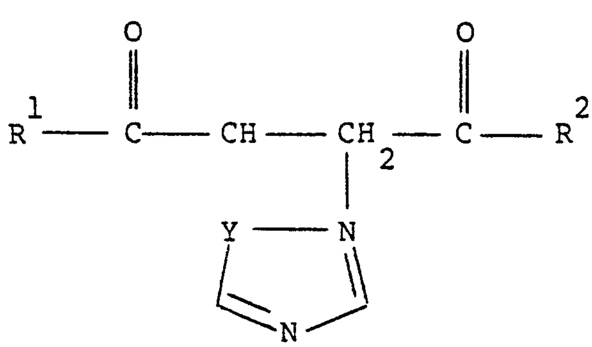

and (e) selectivity reducing the diketone produced by step (d).

In the first stage of the reaction, i.e. stage (a) a convenient way is to carry out the reaction in the presence of a base such as, for example, sodium hydride or a sodium alkoxide.

In the second stage of the reaction, i.e. stage (b) the basic conditions for hydrolysis may be created by adding sodium hydroxide to the reaction mixture.

Stage (c) oxidation may be effected using N-bromo succinimide or 2,3-dichloro-5,6-dicyano-1,4-benzoquinone, but as an alternative a transition metal such as, for example, nickel or palladium on carbon, or a transition metal halide such as, for example, palladium chloride ($PdCl_2$) or a complex thereof with phenyl cyanide of formula $[PdCl_2(C_6H_5CN)_2]$ may be used.

In stage (d) imidazole or 1,2,4-triazole, or a salt thereof, is brought into reaction with the appropriate unsaturated diketone in a high boiling hydro-carbon solvent (e.g. toluene or xylene) at refluxing temperatures. Thus for example 1,2,4-triazole may be reacted with a compound of general formula:

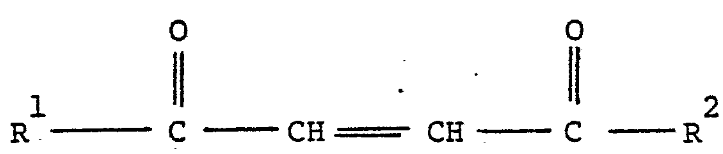

where $R^1$ and $R^2$ are as defined above.

In stage (e) selective reduction of the diketone of general formula:

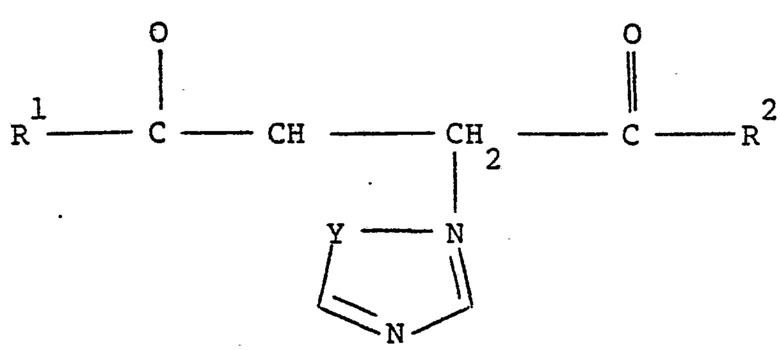

wherein Y, $R^1$ and $R^2$ are as defined above, the diketone or a salt or metal complex thereof, may be brought into reaction with, for example, a metal hydride reducing agent (e.g. lithium aluminium hydride or sodium borohydride) in an inert polar solvent (e.g. diethyl ethyl, water or methanol).

The reduction of the diketone generally involves dissolving the reactants in a solvent such as diethyl ether or tetrahydrofuran (for lithium aluminium hydride reduction) or methanol or water (for sodium borohydride reduction). The temperature at which the reaction may be carried out will depend on the reactants and solvent but generally the reaction mixture is heated under reflux or, if methanol is used, kept at room temperature or, preferably, at $0^oC$ for 1 hour. The reaction product is then isolated by, for example, extraction into a convenient solvent after acidification with a dilute mineral acid. After removal of the solvent in vacuo, the product may be crystallised from a convenient solvent.

Salts, metal complexes, ethers, esters and silyl ethers of the compounds of general formula (I) can be prepared from the latter in known manner. For example, the complexes can be made by reaction the uncomplexed compound with a metal salt in a suitable solvent. When $R^1$ or $R^2$ is substituted phenyl the substituent on the phenyl group can often be changed by methods known in the art. For example, a compound wherein, for example, $R_1$ is phenyl substituted with carboxy, can be prepared from the corresponding compound wherein $R_1$ is phenyl substituted with alkoxycarbonyl, and vice-versa.

If desired stages (a) and (b) can be "telescoped", that is it is possible to make the dione produced by stage (b) without isolation of the intermediate ester produced by stage (a).

The invention further provides a process for producing unsaturated diones comprising, in combination stages (a), (b) and (c), carried out separately with isolation of the individual products, or "telescoped" as described in the previous paragraph.

The invention further provides, as inventive processes in themselves, the individual stages (a), (b) and (c), each in isolation.

The invention is illustrated by the following Example.

EXAMPLE 1

This Example illustrates the preparation of the compound 4-(1,2,4-Triazol-1-yl)-2,2,7,7-tetramethyl-octan-3-ol-6-one having the structure:

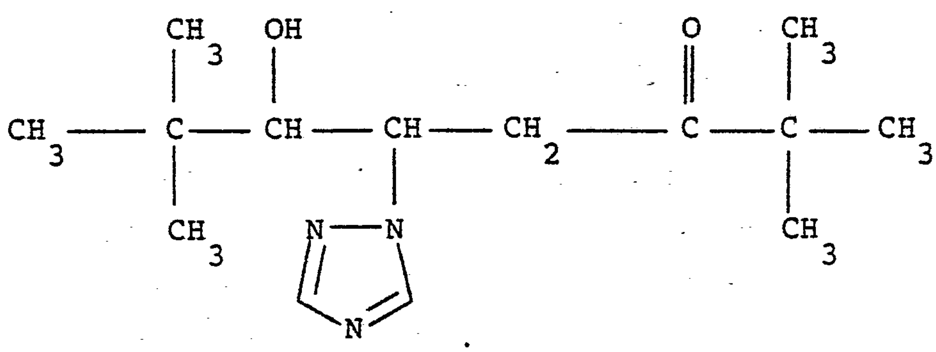

in stages (a), (b), (c), (d) and (e).

Stage (a): Sodium hydride (2.5g, 100%) was suspended in *DMF (250 ml) and methyl pivaloyl acetate (0.2 mol) added dropwise and stirred at room temperature for 7 hours and then stirred at 60°C for 4 hours. After cooling to room temperature the solution was decanted off from the excess sodium hydride. Bromo pinacolone (0.1 mol) in DMF (50 ml) was added to the stirred solution at 0° and the solution stirred at room temperature for 2 hours and at 60°C for 3 hours.

* DMF is dimethyl formamide.

The DMF was removed *in vacuo* and to the residue water was added (150 ml) and neutralised with 2N HCl (2 x 150 ml). The solution was extracted with ether, the organic layer was washed with water and dried over anhydrous sodium sulphate. Removal of the solvent gave 2,2,7,7-tetramethyl-4-carboxymethyl-octan-3,6-dione (yield 90%) as a pale yellow oil.

Stage (b): 2,2,7,7-tetramethyl-4-carboxymethyl-octan-3,6-dione (10.0 g) was warmed on a steam bath with a solution of sodium hydroxide (3.0 g) in water (50 ml) and ethanol (20 ml) for 2 hours. Water (200 ml) was added and the solution extracted with ether (200 ml) washed with water (2 x 150 ml) and the extract dried over anhydrous sodium sulphate. Removal of the solvent gave a pale yellow liquid which was distilled at the oil pump to give 2,2,7,7-tetramethyl-octan-3,6-dione (85%) as a pale yellow liquid b.p. 56-58$^{o}$ (0.1 mm Hg).

Stage (c): 2,2,7,7-tetramethyl-octan-3,6-dione (0.01 mol) and N-bromo succinimide (0.01 mol) were refluxed in $CCl_4$ (30 ml) under a 100w incandescent bulb for 3 hours. The solution was cooled to room temperature and the succinimide filtered off. Removal of the solvent gave an oil which solidified on scratching. Recrystallisation from ethanol gave the title compound (60%) as pale yellow platelets m.p. 102-103$^{o}$.

Stage (d): 2,3,7,7-tetramethyl-oct-4-en-3,6-dione (0.01 mol) and 1,2,4-triazole (0.01 mol), suspended in toluene (100 ml), were refluxed for 24 hours. On cooling to room temperature, the organic layer was washed with water (4 x 100 ml) and dried over sodium sulphate. Removal of the solvent gave 4-(1,2,4-triazol-1-yl)-2,2,7,7-tetramethyl-octan-3,6-dione, m.p. 66-69$^{o}$.

Stage (e): 4-(1,2,4-triazol-1-yl)-2,2,7,7-tetramethyl-octan-3,6-dione (0.02 mol) was dissolved in methanol (50 ml). Sodium borohydride (0.005 mol) was added portionwise at room temperature and the solution kept at $0^{o}C$ for 1 hour. Removal of the methanol gave a white solid which was dissolved in dilute hydrochloric acid (50 ml) and the solution extracted into chloroform (50 ml), washed with saturated sodium bicarbonate (50 ml) and with water (2 x 100 ml) and dried over anhydrous sodium sulphate. Removal of the chloroform gave a colourless oil which was crystallised from petroleum ether/chloroform to give the title compound as a white crystalline solid, m.p. $95-96^{o}$.

1. A process for preparing compounds having the general formula (I):

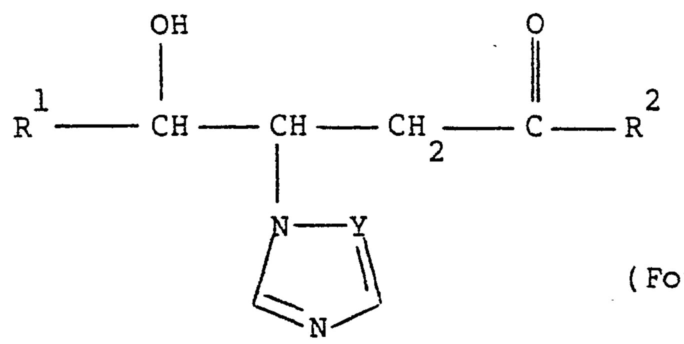

(Formula I)

wherein each of $R^1$ and $R^2$, which may be the same or different is unsubstituted or alkyl-substituted cycloalkyl, unsubstituted or halo-substituted alkyl, or unsubstituted or substituted phenyl, and Y is =N- or =CH-, which comprises the steps of (a) reacting a β-keto ester of formula:

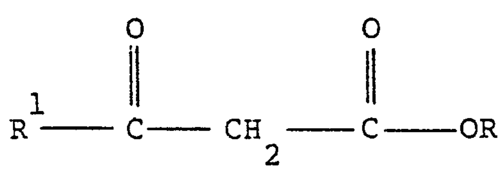

where R is alkyl and especially methyl or ethyl, and $R^1$ is as defined above, with an α-halo ketone of formula:

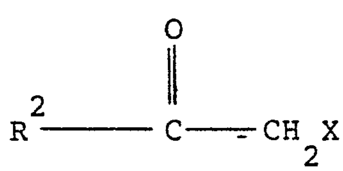

where X is halogen, especially chlorine or bromine, and $R^2$ is as defined above, to produce a diketo-ester of formula:

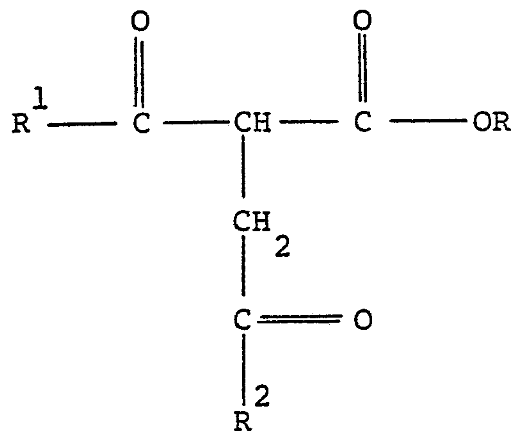

(b) hydrolysing the diketo-ester under basic conditions to give a standard diketone of formula:

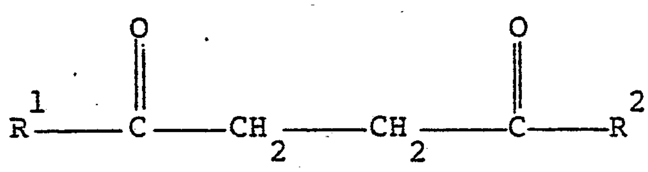

(c) oxidising the saturated diketone to give an unsaturated diketone of formula:

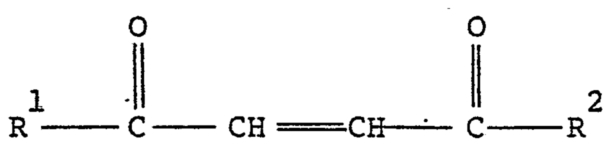

(d) bringing the unsaturated diketone (ene-dione) into reaction with 1,2,4-triazole or imidazole to produce the compound of formula:

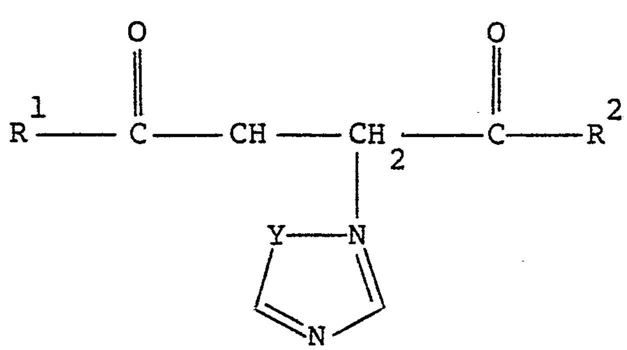

and (e) selectively reducing the diketone produced by step (d).

2. A process as claimed in claim 1 wherein in stage (a) the reaction is carried out in the presence of a base such as, for example, sodium hydride or a sodium alkoxide.

3. A process as claimed in claim 1 or claim 2 wherein in the second stage of the reaction, i.e. stage (b), the basic conditions for hydrolysis are created by adding sodium hydroxide to the reaction mixutre.

4. A process as claimed in any of the preceding claims wherein in stage (c) oxidation is effected using N-bromo succinimide or 2,3-dichloro-5,6-dicyano-1,4-benzoquinone, or a transition metal such as, for example, nickel or palladium on carbon, or a transition metal halide such as, for example, palladium chloride ($PdCl_2$) or a complex thereof with phenyl cyanide of formula $[PdCl_2(C_6H_5CN)_2]$.

5. A process as claimed in any of the preceding claims wherein in stage (d) imidazole or 1,2,4-triazole, or a salt thereof, is brought into reaction with the appropriate unsaturated diketone of formula:

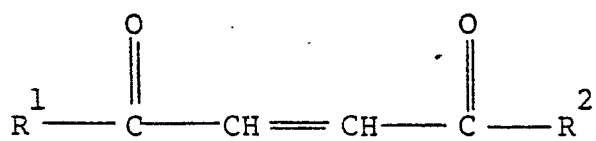

where $R^1$ and $R^2$ are as defined above, in a high boiling hydrocarbon solvent (e.g. toluene or xylene) at refluxing temperatures.

6. A process as claimed in any of the preceding claims wherein in stage (e) selective reduction of the diketone of general formula:

 

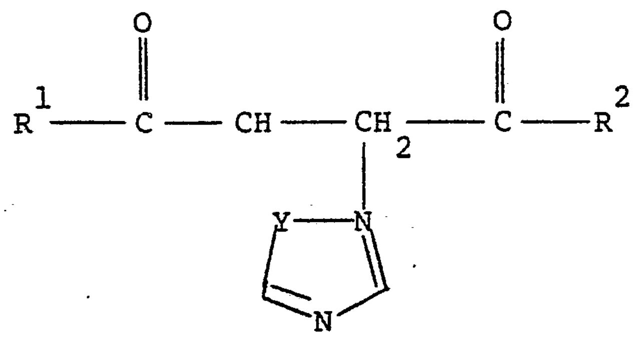

wherein Y, $R^1$ and $R^2$ are as defined above, or a salt or metal complex thereof, is brought into reaction with, for example, a metal hydride reducing agent (e.g. lithium aluminium hydride or sodium borohydride) in an inert polar solvent (e.g. diethyl ethyl, water or methanol).

7. A process as claimed in claim 6 wherein the reduction of the diketone is carried out by dissolving the reactants in a solvent such as diethyl ether or tetrahydrofuran (for lithium aluminium hydride reduction) or methanol or water (for sodium borohydride reduction) and heating the reaction mixture under reflux or, if methanol is used, maintaining it at room temperature or, preferably, at $0^{o}C$, or 1 hour.

8. A process as claimed in claims 6 or 7 wherein the reaction product is isolated by extraction into a solvent after acidification with a dilute mineral acid, removal of the solvent *in vacuo*, and crystallisation of the product from a convenient solvent.

9. A process as claimed in any of the preceding claims wherein a salt, metal complex, ether, ester or silyl ether of a compound of general formula (I) is prepared by reaction of the uncomplexed compound with a metal salt in a suitable solvent.

10. A process as claimed in any of the preceding claims wherein stages (a) and (b) are merged so that the dione produced by stage (b) is made without isolation of the intermediate ester produced by stage (a).

11. A process for producing unsaturated diones comprising, in combination, stages (a), (b) and (c) as defined in any of the preceding claims carried out separately with isolation of the individual products; or merged so as to run consecutively without isolation of the individual products.

12. A process as defined in any of the preceding claims and comprising an individual stage (a), (b) or (c) in isolation.

13. A process according to any of the preceding claims wherein $R^1$ and $R^2$ are t-butyl, R is methyl and X is bromo.

14. A process as defined in any of the preceding claims for the preparation of the compound 4-(1,2,4-triazol-1-yl)-2,2,7,7-tetramethyl-octan-3-ol-6-one having the formula:-

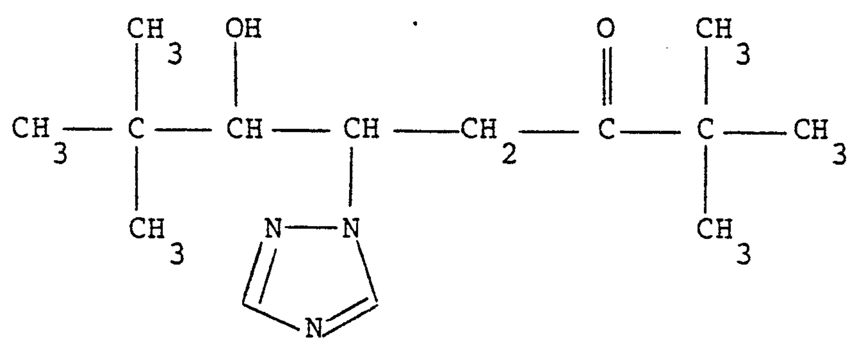

HGHA/bgg
SPEC46